# EUROPEAN PATENT SPECIFICATION

(11) **EP 1 967 214 B1**
(45) Date of publication and mention of the grant of the patent: **06.07.2011**
(21) Application number: 08151885.4
(22) Date of filing: 25.02.2008
(51) Int. Cl.: A61L 9/03, B65D 83/00

(54) **Diffuser device for liquid substances**
Diffusorvorrichtung für flüssige Substanzen
Dispositif de diffuseur pour substances liquides

(30) Priority: 27.02.2007 IT BO20070131
(43) Date of publication of application: 10.09.2008
(73) Proprietor: ZOBELE HOLDING S.P.A., 38100 Trento (IT)
(72) Inventor: Marchetti, Fabio, 38050 Povo (Trento) (IT); Zobele, Franco, 38100 Trento (IT)
(74) Representative: Faggioni, Marco

(56) References cited:
- EP-A- 1 685 856
- WO-A-98/19526
- WO-A-03/086487
- US-A1- 2005 276 584

## Description

The present invention relates to a diffuser device for liquid substances.

As is known, to diffuse scents, insecticide substances or balsamic or medicinal substances into the environment, devices which use systems of emanation by heating are currently used.

Such devices comprise a container with the substance to be released into the environment, which is carried to the outside of the container by means of a porous element, or wick, partly immersed in the liquid substance.

Emanation of the substance in the environment occurs by means of the joule effect, by passing an electric current in a heating element, such as a ceramic element or resistor, located close to or directly in contact with the wick. Said element, heating up, transmits heat to the wick, directly or by heating the surrounding air, promoting evaporation of the substance.

Such devices are usually powered by mains electricity.

In buildings, electrical outlets are usually located on walls and angled in such a way that the slots for insertion of common plug pins are aligned perpendicularly to the floor or parallel with it. Electrical plugs of electrical appliances or electronic equipment are usually connected to the outlets by a cable. Therefore, the angle of the plug relative to the floor does not matter.

In contrast , diffusers for liquid substances normally have the plug directly attached to the device. This may bring problems considering the fact that such diffusers must be kept in a vertical position, since they contain liquid substances which could leak out or may not soak the wick correctly, preventing diffusion of the substance.

To overcome said disadvantage, current diffuser devices have electrical plugs which can be angled, that is to say, electrical plugs which can turn through ninety degrees so that they can be inserted both in electrical outlets with slots vertical relative to the floor and in electrical outlets with slots horizontal relative to the floor, allowing the diffuser to be kept in the same upright position.

Diffuser devices having a rotatable plug of the above said type are, for example, disclosed in EP-1685856 and WO-03/086487.

However, such a mechanism brings disadvantages because it involves sliding contacts which are delicate and complex to make. Continuous rotation of the plug and the consequent sliding of the contacts causes them to wear and deteriorate.

Moreover, common diffuser devices also have another disadvantage. Some devices have no system for adjusting the intensity of emanation. Often, the portion of wick outside the tank and the heating element are closed in a small space having a single grille or a small hole from which the product to be evaporated comes out. In contrast, other devices have systems for varying the intensity of diffusion but they are based on just a few adjusting levels which therefore allow small, not always optimum adjustments. Such systems are generally produced using a sliding wall or a small window which closes the space in which the wick soaked with liquid is present. The window gap determines the intensity of emanation of the substance. However, the openings which can be obtained do not always correspond to the desired level of intensity of emanation.

The aim of the present invention is to provide a diffuser device able to eliminate the above-mentioned disadvantages.

In particular, the present invention has for an aim to propose a diffuser device which has a simple structure and which can be connected both to a vertically angled electrical outlet and to a horizontally angled electrical outlet.

The present invention also has for an aim to provide a diffuser device which allows continuous and gradual adjustment of the intensity of emanation.

Accordingly the present invention provides a diffuser device for liquid substances comprising the characteristics defined in the main claim 1. Additional features are defined in the dependent claims. The present invention is described below with reference to the accompanying drawings, which illustrate a non-limiting embodiment, in which:
- Figure 1 is a perspective rear view of a diffuser device for liquid substances in accordance with the present invention, in a first operating position;
- Figure 2 is a perspective rear view of a diffuser device for liquid substances in accordance with the present invention, in a second operating position;
- Figure 3 is a perspective rear view of the device of Figure 1;
- Figure 4 is a front view of a first embodiment of the device of Figure 1 illustrating a first adjustment and with some parts removed for clarity;
- Figure 5 is a view of the device in accordance with the first embodiment of Figure 4, illustrating a second adjustment and with some parts removed for clarity;
- Figure 6 is a plan view of the device illustrated in Figure 2;
- Figure 7 is a view of a second embodiment of the device in accordance with the present invention;
- Figure 8 is a view of a component of the device illustrated in Figure 7;
- Figure 9 is a view of an enlarged portion of the component illustrated in Figure 8, before use of the device made in accordance with the present invention;
- Figure 10 is a view of an enlarged portion of the component illustrated in Figure 8, after use of the device made in accordance with the present invention.

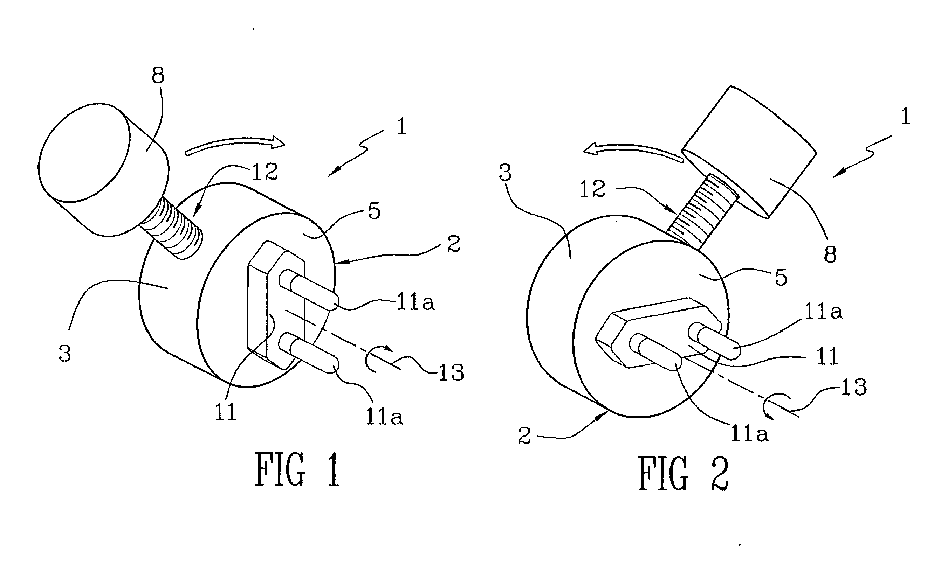

With reference to Figures 1 and 2, the numeral 1 denotes as a whole a diffuser device for liquid substances.

The device 1 comprises a supporting structure 2, substantially box-shaped and having, for example, a circular, square or generic polygonal shape depending on the appearance of the diffuser.

The supporting structure 2 comprises a lateral surface 3 forming a portion of space 4, closed at a first end by a first front surface 5 and at a second end by a second front surface 6, both suitably shaped so as to join correctly with the lateral surface 3.

In a precise position indicated below, the lateral surface 3 has a hole 7, preferably threaded, for insertion of a tank 8 containing liquid to be diffused.

The tank 8, usually made of shatterproof glass or transparent material, has at least partly inside it, and in particular close to its first end 8b, an element 9, usually porous, which absorbs the liquid and carries it to the outside. In particular, the porous element 9 is a wick having a first end 9d constantly in contact with the liquid and a second end 9a located close to or in contact with a heating element 10, inside the device 1.

The second end 9a of the wick 9 has a base surface 9b giving onto the heating element 10.

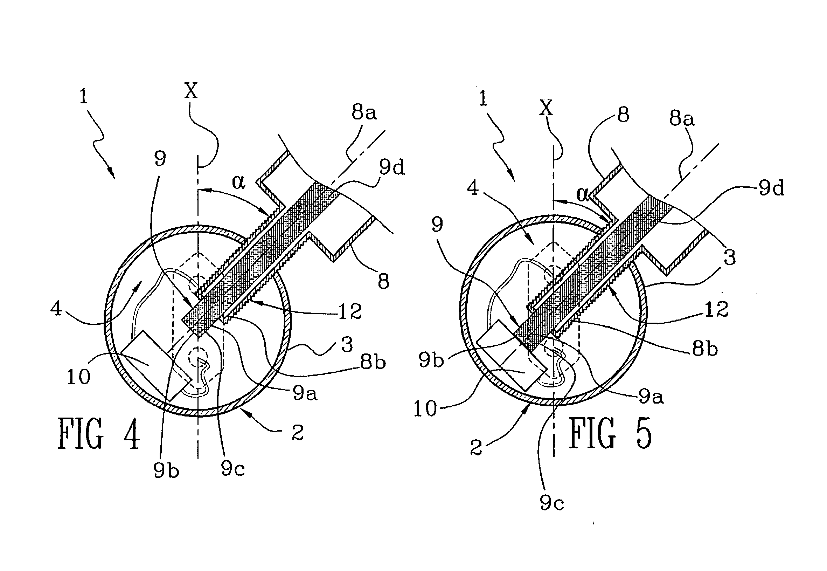

In a first embodiment, illustrated in Figures 4 and 5, the porous element 9 partly projects from the tank 8.

In particular, the first end 9d is completely immersed in the liquid to be emanated, whilst the second end 9a is completely outside the tank 8. In this way, the active evaporation surface is formed by both the lateral surface 9c of the wick and the base surface 9b of the second end 9a.

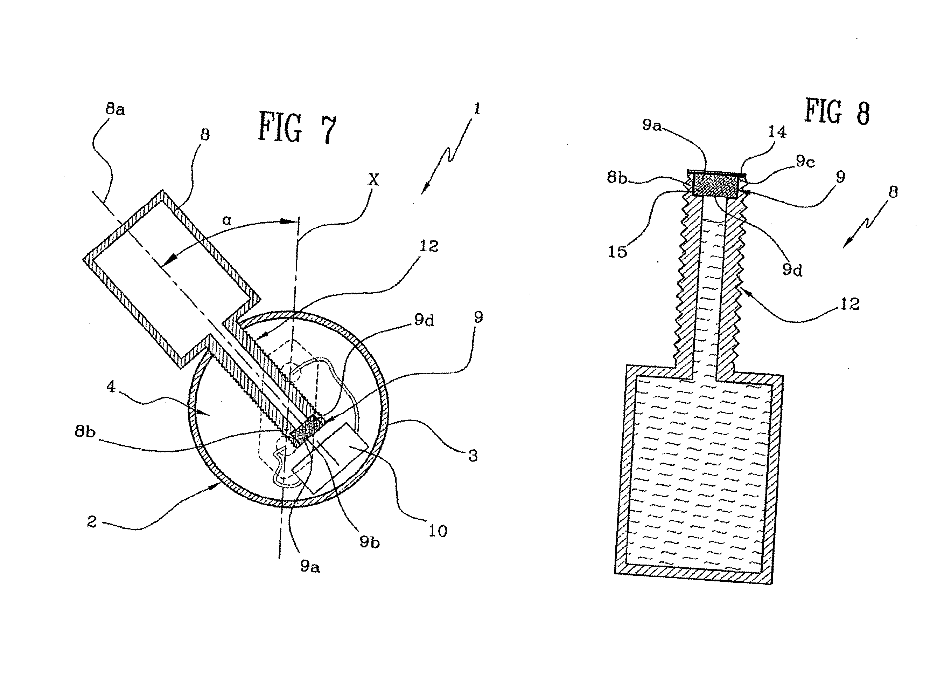

In a second embodiment, illustrated in Figure 7, the porous element 9 is completely contained in the tank 8, in a cavity 15 made close to the first end 8b of the tank 8. In particular, the base surface 9b of the wick is aligned with the base surface of the first end 8b of the tank 8. In this way, the base 9b is the only surface of the wick 9 giving onto the outside of the tank 8 and acts as the evaporation surface. The lateral surface 9c is completely inside the tank 8, and the base surface of the first end 9d of the wick is in direct contact with the liquid to be diffused.

The heating element 10 is a ceramic element comprising, inside it, an electrical circuit which heats the second end 9a of the wick 9, promoting evaporation of the liquid substance with which the wick is soaked.

Both the heating element 10 and a variable portion of the tank 8 are contained in the space 4 delimited by the lateral surface 3 and by the two front surfaces 5 and 6.

Usually at least two pins 11a of an electrical plug 11 project from the first front surface 5, the plug being designed to connect the device 1, and in particular the heating element 10, to the mains electricity so as to power the inner heating element.

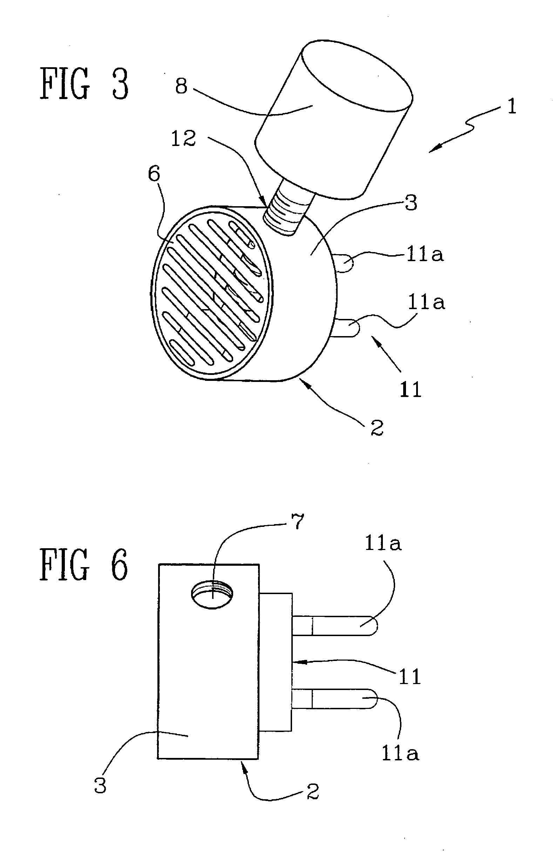

In contrast, the second front surface 6 preferably has a surface equipped with a grille or with holes or slots made in it, as shown in Figure 3, to allow diffusion of the evaporated substance in the environment.

The tank 8 has a portion 12 threaded on the outside for screwing into the hole 7. The thread allows continuous and gradual adjustment of the distance between the wick 9 and the heating element 10, between a maximum distance position, illustrated in Figure 4, in which substance emission is at a minimum, and a minimum distance position, with contact between the two elements in the case of the first embodiment, as illustrated in Figure 5, in which substance emission is at a maximum. Many intermediate adjusting positions are therefore possible, which the user can control according to the desired intensity of emanation, by simply screwing in or unscrewing the tank 8 as required, thus regularly increasing or reducing the distance between the base surface 9b of the second end 9a of the wick 9 and the heating element 10.

Before insertion in the diffuser device 1, the tank 8 is closed to prevent evaporation of the liquid in the air and to protect the porous element 9. In the first embodiment the tank 8 has a screw or pressure cap, which may be reclosable, which is screwed onto the threaded outer portion 12 on the outside of the tank 8.

In contrast, in the second embodiment illustrated in Figure 8, the tank 8 is usually equipped with closing systems 14 which cannot be replaced, such as a heat-soluble film, sealed or glued to the tank, which is gradually dissolved by the closeness of the heating element 10, or a simple film which can be torn off, also sealed or glued to the tank, or alternatively an element which breaks when the tank 8 is inserted in the diffuser device 1.

Alternatively, in the second embodiment the tank 8 may have a reclosable screw or pressure cap, like that described with reference to the first embodiment.

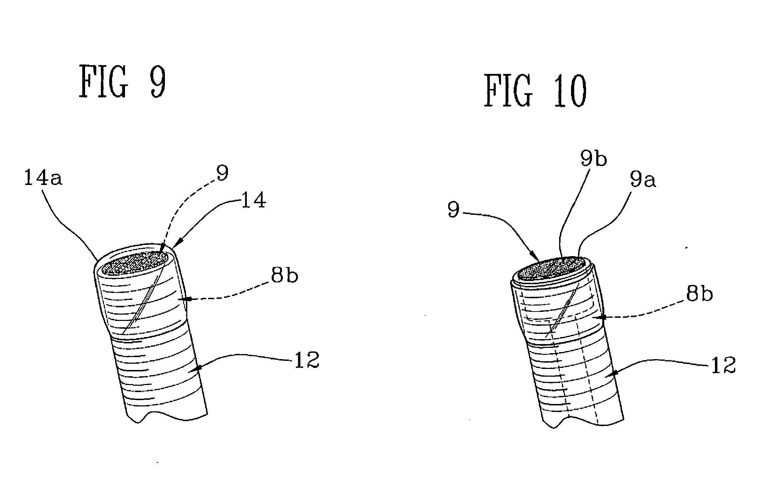

An example of a closing system using a heat-soluble film is illustrated in Figure 9 and 10, which show a covering cap 14a, covering the entire first end 8b of the tank 8 and protecting the porous element 9 until the device 1 is first used.

This covering cap 14a is made of a polyamide gelatinising agent, formulated for use with moderately polar organic substances, having a melting temperature of less than 100°C, approximately around 80°C. The cap 14a is applied by partly immersing the first end 8b of the tank 8 in the polyamide substance. Once it has cooled, the polyamide solidifies, creating a waterproof waxy cap which is thermally stable with the passage of time, preserving the integrity of the porous element 9 without altering its mechanical properties and without blocking its micro-pores. Moreover, the cap 14a prevents evaporation and dispersion of the liquid substance, does not alter its chemical composition and is not toxic for humans.

When the diffuser device 1 is activated, the heating element 10 gradually increases its temperature until it reaches temperatures of around 100°C, at which the liquid contained in the tank 8 begins to evaporate. The closeness of the heating element 10 therefore produces, already in the first few minutes of use of the diffuser device 1, the gradual and definitive melting of the cap 14a substance, thus leaving the porous element 9 uncovered opposite the heating element 10.

Consequently the liquid substance begins evaporating.

It should be noticed that melting of the substance forming the cap 14a does not interfere with the speed of evaporation, nor does it cause any damage to the electrical components of the heating element 10.

As already indicated, and visible in Figures 4, 5 and 7, the position of the hole 7 is such that the longitudinal axis 8a of the porous element 9, and therefore of the tank 8, forms with the plane X containing the plug 11 pins 11a an angle α preferably of between 15° and 75°, and more preferably of between 30° and 60°.

In particular, said angle α is advantageously between 40° and 50°.

In this way, the device 1 can be connected to the mains electricity supply whatever the angle of the electrical outlet on the wall. Therefore, if the slots for insertion of the pins are aligned perpendicularly relative to the floor, the diffuser device 1 is positioned as illustrated in Figure 1. If instead the outlet on the wall is parallel with the floor, a simple 90° rotation of the entire device 1 about an axis 13 at a right angle to the front surfaces 5 and 6, as indicated by the arrow in Figure 1, brings the diffuser into the position illustrated in Figure 2. It may be seen how the tank always remains at the same angle relative to a vertical plane.

The accompanying drawings show the tank 8 positioned in the upper part of the device 1, angled in such a way that the second end 9a of the wick 9 points downwards and the entire wick 9 is positioned above the heating element 10.

Only in the case of the first embodiment illustrated in Figure 4 and 5, the tank 8 may also be positioned in the lower part of the device 1, angled in such a way that the second end 9a of the wick 9 points upwards, and therefore the entire wick 9 is positioned below the heating element 10.

This is possible thanks to the fact that in this embodiment the wick is long enough to draw liquid from the bottom of the tank even when the liquid is running out.

The relative angle between the tank or wick and the plane containing the plug pins allows a diffuser device to be obtained which can be connected to a generic wall outlet having slots for insertion of the pins which may be perpendicular to or parallel with the floor, the device having a fixed plug without any sliding contacts. Therefore, wear on the electrical contacts is significantly reduced compared with that of sliding contacts in the prior art. Moreover, a plug which is fixed relative to the entire device, that is to say, a plug which cannot rotate independently of the rest of the device, also simplifies the structure and reduces production costs.

Adjustment by screwing the tank more or less completely into the hole in the supporting structure allows more gradual adjustment of the scent, which is more controlled at the user's discretion.

The invention described is susceptible of industrial application and may be modified and adapted in several ways without thereby departing from the scope of the inventive concept. Moreover, all details of the invention may be substituted by technically equivalent elements.

## Claims

1. A diffuser device for liquid substances comprising:
- a tank (8) containing a liquid to be diffused;
- a supporting structure (2) comprising a lateral surface (3) having a hole (7) for insertion of the tank (8) and a first front surface (5) from which a plug (11) projects, the plug comprising two pins (11a) for connecting the diffuser device (1) to an electrical outlet;
- a heating element (10) inside the supporting structure (2), electrically connected to the plug (11) pins;
- a porous element (9) at least partly inserted in the tank (8), for carrying the liquid from the tank (8) to the heating element (10);
**characterised in that**
- said plug (11) is a fixed plug without any sliding contacts;
- the porous element (9) is set at an angle to a plane (X) containing the plug pins (11a) so that the longitudinal axis (βa) of the porous element (9) forms with said plane an angle (α) of between 15° and 75°;
- the tank (8) has a portion which is threaded on the outside (12) for screwing into the threaded hole (7), so as to regularly adjust the distance between the porous element (9) and the heating element (10) between a maximum distance position corresponding to minimum intensity diffusion, and a minimum distance position corresponding to maximum intensity diffusion.

2. The device according to claim 1, wherein the angle (α) is between 30° and 60°, preferably between 40° and 50°.

3. The device according to claim 1) or 2), wherein the porous element (9) is a wick having a first end (9d) constantly in contact with the liquid contained in said tank (8) and a second end (9a) located inside the device (1) and close to or in contact with said heating element (10).

4. The device according to claim 3), wherein the second end of the wick (9) has a base surface (9b) giving onto the heating element (10).

5. The device according to claim 4), wherein the porous element (9) has a first end (9d) completely inserted in the tank (8) and a second end (9a) completely outside the tank (8).

6. The device according to claim 4), wherein the porous element (9) is completely contained in the tank (8) in such a way that only a base surface (9b) gives onto the outside of the tank (8), aligned with an outer surface of the tank (8), and acting as an evaporation surface.

7. The device according to any one of the foregoing claims, wherein the heating element (10) is made of ceramic.

8. The device according to claim 7), wherein the heating element (10) comprises an internal electrical resistor.

9. The device according to claim 6), wherein the porous element (9) is protected by a closing system (14) which is removable and cannot be replaced.

10. The device according to claim 9), wherein the closing system (14) comprises a covering made of a heat-soluble substance, or of a film which can be removed by tearing it off or of an element that can be broken.

## Patentansprüche

1. Diffusorvorrichtung für flüssige Substanzen, mit:
- einem Tank (8), der eine zu zerstreuende Flüssigkeit enthält;
- einer Tragstruktur (2), die eine seitliche Oberfläche (3) mit einem Loch (7) umfasst, um den Tank (8) einzusetzen, und eine erste Frontfläche (5), von der ein Stecker (11) vorsteht, wobei der Stecker zwei Stifte (11a) zum Anschließen der Diffusorvorrichtung (1) an einen elektrischen Auslass umfasst;
- einem Beheizungselement (10) innerhalb der Tragstruktur (2), die elektrisch mit den Stiften des Steckers (11) verbunden ist;
- einem porösen Element (9), das zumindest teilweise in den Tank (8) eingesetzt ist, um die Flüssigkeit von dem Tank (8) zu dem Beheizungselement (10) zu transportieren;
**dadurch gekennzeichnet, dass**
- der genannte Stecker (11) ein feststehender Stecker ohne irgendwelche verschiebliche Kontakte ist;
- das poröse Element (9) unter einem Winkel zu einer Ebene (X) angeordnet ist, die die Stifte (11a) des Steckers enthält, so dass die Längsachse (8a) des porösen Elements (9) mit der genannten Ebene einen Winkel (α) zwischen 15° und 75° bildet;
- der Tank (8) einen Abschnitt aufweist, der auf der Außenseite (12) mit Gewinde versehen ist, zum Einschrauben in das mit Gewinde versehene Loch (7), um den Abstand zwischen dem porösen Element (9) und dem Beheizungselement (10) regelmäßig einzustellen, zwischen einer Position mit maximalem Abstand, die einer minimalen Diffusionsintensität entspricht, und einer Position mit minimalem Abstand, die einer maximalen Diffusionsintensität entspricht.

2. Vorrichtung nach Anspruch 1, **dadurch gekennzeichnet, dass** der Winkel (α) zwischen 30° und 60° liegt, vorzugsweise zwischen 40° und 50°.

3. Vorrichtung nach Anspruch 1 oder 2, **dadurch gekennzeichnet, dass** das poröse Element (9) ein Docht ist, der ein erstes Ende (9d) aufweist, das konstant mit der Flüssigkeit in Kontakt steht, die in dem genannten Tank (8) enthalten ist, und ein zweites Ende (9a), das innerhalb der Vorrichtung (1) angeordnet ist und nahe an oder in Kontakt mit dem genannten Beheizungselement (10) ist.

4. Vorrichtung nach Anspruch 3, **dadurch gekennzeichnet, dass** das zweite Ende des Dochts (9) eine Basisoberfläche (9b) aufweist, die zu dem Beheizungselement (10 gerichtet ist.

5. Vorrichtung nach Anspruch 4, **dadurch gekennzeichnet, dass** das poröse Element (9) ein erstes Ende (9d) aufweist, das vollständig in den Tank (8) eingesetzt ist, und ein zweites Ende (9a), das vollständig außerhalb des Tanks (8) liegt.

6. Vorrichtung nach Anspruch 4, **dadurch gekennzeichnet, dass** das poröse Element (9) vollständig innerhalb des Tanks (8) auf eine solche Weise enthalten ist, dass nur eine Basisoberfläche (9b) zur Außenseite des Tanks (8) weist, ausgerichtet mit einer äußeren Oberfläche des Tanks (8), und als Verdampfungsoberfläche wirkt.

7. Vorrichtung nach einem der vorangehenden Ansprüche, **dadurch gekennzeichnet, dass** das Beheizungselement (10) aus Keramik besteht.

8. Vorrichtung nach Anspruch 7, **dadurch gekennzeichnet, dass** das Beheizungselement (10) einen internen elektrischen Widerstand umfasst.

9. Vorrichtung nach Anspruch 6, **dadurch gekennzeichnet, dass** das poröse Element (9) von einem Verschlusssystem (14) geschützt ist, das abnehmbar ist und nicht erneut aufgesetzt werden kann.

10. Vorrichtung nach Anspruch 9, **dadurch gekennzeichnet, dass** das Verschlusssystem (14) eine Abdeckung umfasst, die aus einer wärmelöslichen Substanz besteht, oder aus einem Film, der durch Abziehen entfernt werden kann, oder aus einem Element, das zerbrochen werden kann.

## Revendications

1. Dispositif diffuseur pour substances liquides comprenant :
- un réservoir (8) contenant un liquide destiné à être diffusé ;
- une structure de support (2) comprenant une surface latérale (3) comportant un trou (7) pour insertion du réservoir (8) et une première surface avant (5) d'où saillit une fiche d'alimentation électrique (11), la fiche comprenant deux broches (11a) pour connexion du dispositif diffuseur (1) à une prise de courant ;
- un élément de chauffage (10) à l'intérieur de la structure de support (2), relié électriquement aux broches de la fiche d'alimentation électrique (11) ;
- un élément poreux (9) inséré, au moins en partie, dans le réservoir (8), pour transporter le liquide du réservoir (8) à l'élément de chauffage (10) ;
**caractérisé en ce que**
- ladite fiche d'alimentation électrique (11) est une fiche d'alimentation électrique (11) fixe sans contacts glissants ;
- l'élément poreux (9) est placé à un angle par rapport à un plan (X) contenant les broches (11a) de fiche d'alimentation électrique de manière que l'axe longitudinal (8a) de l'élément poreux (9) forme, avec ledit plan, un angle (α) compris entre 15° et 75° ;
- le réservoir (8) comporte une partie qui est enfilée sur l'extérieur (12) pour vissage dans le trou taraudé (7), afin d'ajuster régulièrement la distance entre l'élément poreux (9) et l'élément de chauffage (10) entre une position de distance maximale correspondant à une diffusion d'intensité minimale, et une position de distance minimale correspondant à une diffusion d'intensité maximale.

2. Dispositif selon la revendication 1, dans lequel l'angle (α) est compris entre 30° et 60°, de préférence entre 40° et 50°.

3. Dispositif selon la revendication 1 ou 2, dans lequel l'élément poreux (9) est une mèche ayant une première extrémité (9d) constamment en contact avec le liquide contenu dans ledit réservoir (8) et une seconde extrémité (9a) située à l'intérieur du dispositif (1) et proche de, ou en contact avec, ledit élément de chauffage (10).

4. Dispositif selon la revendication 3, dans lequel la seconde extrémité de la mèche (9) comporte une surface de base (9b) donnant sur l'élément de chauffage (10).

5. Dispositif selon la revendication 4, dans lequel l'élément poreux (9) a une première extrémité (9d) complètement insérée dans le réservoir (8) et une seconde extrémité (9a) complètement à l'extérieur du réservoir (8).

6. Dispositif selon la revendication 4, dans lequel l'élément poreux (9) est complètement contenu dans le réservoir (8) de manière que seule une surface de base (9b) donne sur l'extérieur du réservoir (8), en alignement avec une surface externe du réservoir (8), et servant de surface d'évaporation.

7. Dispositif selon l'une quelconque des revendications précédentes, dans lequel l'élément de chauffage (10) est en céramique.

8. Dispositif selon la revendication 7, dans lequel l'élément de chauffage (10) comprend une résistance électrique interne.

9. Dispositif selon la revendication 6, dans lequel l'élément poreux (9) est protégé par un système de fermeture (14) qui est amovible et ne peut être remis en place.

10. Dispositif selon la revendication 9, dans lequel le système de fermeture (14) comprend une couverture faite d'une substance soluble à la chaleur, ou d'un film qui peut être ôté par arrachage ou d'un élément qui peut être rompu.
